# EUROPEAN PATENT SPECIFICATION

(11) **EP 0 989 850 B1**
(45) Date of publication and mention of the grant of the patent: **03.11.2004**
(21) Application number: 98903378.2
(22) Date of filing: 16.01.1998
(51) Int. Cl.: A61K 31/425, A61P 25/00

(54) **USE OF PRAMIPEXOLE IN THE TREATMENT OF RESTLESS LEGS SYNDROME**
VERWENDUNG VON PRAMIPEXOL ZUR BEHANDLUNG VON RESTLESS-LEGS-SYNDROM
UTILISATION DU PRAMIPEXOLE POUR LE TRAITEMENT DU SYNDROME DES JAMBES SANS REPOS

(30) Priority: 17.01.1997 DE 19701619
(43) Date of publication of application: 05.04.2000
(73) Proprietor: PHARMACIA & UPJOHN COMPANY, Kalamazoo, Michigan 49001 (US); Boehringer Ingelheim Pharma GmbH & Co.KG, 55218 Ingelheim am Rhein (DE)
(72) Inventor: OERTEL, Wolfgang, H., D-35282 Ranschenberg (DE); MEIER, Dieter, D-65185 Wiesbaden (DE); GOMEZ-MANCILLA, Baltazar, Portage, MI 49024 (US); MONTPLAISIR, J., H. du Sacre-Coeur de Montreal, Montreal, Quebec (CA)
(74) Representative: Perry, Robert Edward
(86) International application number: PCT/US1998/000216
(87) International publication number: WO 1998/031362

(56) References cited:
- EP-A- 0 186 087
- WO-A-96/18395
- P.M. BECKER ET AL.: "Dopaminergic agents in restless legs syndrome and periodic limb movements of sleep: response and complications of extended treatment in 49 cases." SLEEP, vol. 16, no. 8, 1993, pages 713-716, XP002064709
- J. STAEDT ET AL.: "Nächtliches Myoklonie-syndrom und restless-legs-syndrom-Übersicht und Fallbeschreibung." FORTSCHR. NEUROL. PSYCHIATR., vol. 62, no. 3, 1994, pages 88-93, XP002064710

## Description

### FIELD OF THE INVENTION

The present invention relates to the use of pramipexole in the treatment of restless leg syndrome.

### BACKGROUND OF THE INVENTION

Restless leg syndrome (RLS) is a neurosensorimotor disorder with parestethesias, sleep disturbances and, in most cases, periodic limb movements of sleep (PLMS).

Pramipexole is a dopamine-D₃/D₂ agonist, the synthesis of which is described in EP-A-0186087. It is known primarily for the treatment of schizophrenia and Parkinson's disease.

DE-A-3843227 discloses that pramipexole lowers the plasma level of prolactin. It also discloses the use of pramipexole in the treatment of drug dependency. DE-A-3933738 discloses that pramipexole can be used to decrease abnormal high levels of thyroid stimulating hormone.

US-A-5112842 discloses the transdermal administration of pramipexole and transdermal systems containing it.

WO94/13287 describes pramipexole as an antidepressant agent, while WO96/19078 discloses the neuroprotective effects of pramipexole.

### SUMMARY OF THE INVENTION

The present invention is based on the surprising discovery that pramipexole is useful in the treatment of RLS. Acccording to the invention, pramipexole, by which is meant 2-amino-6-n-propylamino-4,5,6,7-tetrahydrobenzothiazole, its (-)-enantiomer and pharmacologically acceptable salts thereof, especially (-)-2-amino-6-n-propylamino-4,5,6,7-tetrahydrobenzothiazole dihydrochloride (H₂O), is used to prepare a medicament for treating restless legs syndrome.

### DESCRIPTION OF THE INVENTION

2-Amino-6-n-propyl-amino-4,5,6,7-tetrahydrobenzothiazole, particularly the (-)-enantiomer thereof, and the pharmacologically acceptable acid addition salts thereof, can be given for treating RLS. The form of conventional galenic preparations consists essentially of an inert pharmaceutical carrier and an effective dose of the active substance; e.g., plain or coated tablets, capsules, lozenges, powders, solutions, suspensions, emulsions, syrups or suppositories.

Preferred are tablets comprising 0.88 (0.125), 0.18 (0.25), 0.7 (1.0), 0.88 (1.25) and 1.1 (1.5) mg of pramipexole base (mg pramaipexole 2HCl), respectively, and further comprising mannitol, maize starch, colloidal silica, povidone and magnesium stearate as excipients.

The effective dose range is 0.001 to 10.0 mg/day per patient, preferably between 0.001 and 6, more preferably between 0.01 to 6, and most preferably between 0.75 and 4.5 mg/day per patient p.o. In addition to being administered by the oral or intravenous route, pramipexole may also be administered transdermally or by inhalation.

Dosages should be increased gradually, from a starting dose of about 0.264 mg of base per day and then increased every 5-7 days. Providing patients do not experience intolerable side-effects, the dosage should be titrated to achieve a maximal therapeutic effect.

### PILOT STUDY

10 patients with RLS were treated with pramipexole in a cross-over design. The patients received up to 1.5 mg a day of pramipexole over 4 weeks. After the first treatment period, there was a two week wash-out period and an additional 4-week treatment period. Since the symptoms of RLS are quite obvious, their improvement from treatment was obvious to the investigator.

## Claims

1. Use of a compound selected from 2-amino-6-n-propylamino-4,5,6,7-tetrahydrobenzothiazole, the (-)-enantiomer thereof, and pharmacologically acceptable salts thereof, to prepare a medicament for treating restless legs syndrome.

2. The use of claim 1, wherein the compound is pramipexole, its dihydrochloride, or its dihydrochloride hydrate.

3. The use of claim 2, wherein the dose of pramipexole is 0.01-10.0 mg/day.

## Patentansprüche

1. Verwendung einer Verbindung, die aus 2-Amino-6-npropylamino-4,5,6,7-tetrahydrobenzothiazol, dem (-)-Enantiomer desselben und pharmakologisch akzeptablen Salzen desselben ausgewählt ist, zur Herstellung eines Medikaments zur Behandlung des Restless-Legs-Syndroms.

2. Verwendung nach Anspruch 1, wobei die Verbindung Pramipexol, dessen Dihydrochlorid oder dessen Dihydrochloridhydrat ist.

3. Verwendung nach Anspruch 2, wobei die Dosis von Pramipexol 0,01 - 10,0 mg/Tag beträgt.

## Revendications

1. Utilisation d'un composé choisi entre le 2-amino-6-n-propylamino-4,5,6,7-tétrahydrobenzothiazole, son énantiomère (-) et ses sels pharmacologiquement acceptables, pour la préparation d'un médicament destiné au syndrome des jambes sans repos.

2. Utilisation suivant la revendication 1, dans laquelle le composé est le pramipexole, son dichlorhydrate ou son hydrate de dichlorhydrate.

3. Utilisation suivant la revendication 2, dans laquelle la dose de pramipexole est comprise dans l'intervalle de 0,01 à 10,0 mg/jour.
